(19) Europäisches Patentamt
European Patent Office
Office européen des brevets

(11) Publication number: **0 249 883 B1**

(12) **EUROPEAN PATENT SPECIFICATION**

(45) Date of publication of patent specification: **16.09.92**

(51) Int. Cl.5: **C07C 17/38**, C07C 25/02, B01J 29/18, B01J 29/28

(21) Application number: **87108422.4**

(22) Date of filing: **11.06.87**

The file contains technical information submitted after the application was filed and not included in this specification

(54) **Method for adsorptive separation of dichlorotoluenes.**

(30) Priority: **18.06.86 JP 140212/86**
**25.06.86 JP 147051/86**

(43) Date of publication of application:
**23.12.87 Bulletin 87/52**

(45) Publication of the grant of the patent:
**16.09.92 Bulletin 92/38**

(84) Designated Contracting States:
**CH DE FR GB IT LI NL**

(56) References cited:
**EP-A- 0 199 212**
**GB-A- 2 166 734**

(73) Proprietor: **HODOGAYA CHEMICAL COMPANY, LIMITED**
**4-2, Toranomon 1-chome Minato-ku**
**Tokyo 105(JP)**

(72) Inventor: **Kaneshiki, Toshitaka**
**No. 26-14, Naka-cho Itabashi-ku**
**Tokyo(JP)**
Inventor: **Haneda, Tadayoshi**
**No. 26-5, Higashi-araku Ohtuki-machi**
**Koriyama-shi Fukushima-ken(JP)**
Inventor: **Kanno, Sueo**
**No. 26-14, Yokotuka**
**Koriyama-shi Fukushima-ken(JP)**
Inventor: **Hane, Yuichi**
**No. 2-12-9, Hayama**
**Koriyama-shi Fukushima-ken(JP)**
Inventor: **Endo, Toshiyuki**
**No. 27, Aza-Okubo Tadano**
**Ose-machi Koriyama-shi Fukushima-ken(JP)**
Inventor: **Abe, Yoshihiko**
**No. 25, Aza-Mizuguchi Kubota**
**Fukuyama-machi Koriyama-shi**
**Fukushima-(JP)**

(74) Representative: **Wächtershäuser, Günter, Dr.**
**Tal 29**
**W-8000 München 2(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to a method for selectively separating 2,6-dichlorotoluene from a mixture of dichlorotoluene (hereinafter referred to simply as DCT) isomers by means of a zeolite adsorber.

2,6-DCT is an important intermediate useful for the production of agricultural chemicals, medicines, dyestuffs, etc.

A mixture of DCT isomers is produced by chlorination of toluene or monochlorotoluene. The boiling points of the respective isomers are very close to one another, and it is very difficult to separate 2,6-DCT from the mixture by rectification. Therefore, as an industrial method, it is produced by dichlorination of p-toluene sulfonic acid, followed by desulfonation.

Further, US-4,254,062 and JP-A-199,642/1984 disclose a method for adsorptive separation of 2,6-DCT from a mixture of DCT isomers by means of faujasite type zeolite.

However, it is hardly possible to obtain 2,6-DCT of a high purity by the method for the production from p-toluene sulfonic acid. Further, this method is not economical. On the other hand, the latter method for the adsorptive separation by means of a zeolite is designed to separate and recover 2,6-DCT as an extract component from the mixture of DCT isomers. However, the adsorption of 2,6-DCT by faujasite type zeolite is not sufficient, and it is practically impossible to separate and recover 2,6-DCT in high purity. This method also has a drawback that 2,6-DCT can not be separated and recovered unless the adsorptive separation is conducted in the presence of a substituted benzene compound.

GB-A-2 166 734 discloses a method for the separation of an isomeric mixture of dichlorotoluenes using a zeolite of the ZSM-5 type as adsorber. The desired isomers are concentrated and separated by using an eluent.

Under the circumstances, the present inventors have conducted extensive research for a method of effectively recovering by adsorptive separation 2,6-DCT in high purity from a mixture of DCT isomers. As a result, they have found specific catalysts which are capable of selectively separating 2,6-DCT as a non-adsorbed component. The present invention has been accomplished on the basis of this discovery.

The zeolites to be used in the present invention are well-known as isomerization catalysts. As an example for their application to adsorptive separation, an application to alkylbenzenes or phenols is known, but no application to the adsorptive separation of DCT isomers is known.

Namely, the present invention provides a method for selectively separating 2,6-dichlorotoluene from a mixture of dichlorotoluene isomers by means of a zeolite adsorber, characterized in that a zeolite selected from the group consisting of ZSM-8 zeolite, ZSM-ll zeolite, ZSM-2l zeolite, ZSM-35 zeolite, Zeta-l zeolite, Zeta-3 zeolite and TPZ-3 zeolite, is used as the adsorber to selectively separate 2,6-dichlorotoluene as a non-adsorbed component.

Now, the present invention will be described in detail with reference to the preferred embodiments.

In the accompanying drawing, Figure l is a break through curve of an adsober showing the amount of 2,6-DCT effluent till the break through point of the zeolite of the present invention when a mixture of DCT isomers was subjected to adsorptive separation by means of the zeolite.

The zeolite to be used in the present invention is an adsorber having an extremely peculiar characteristic such that it does not adsorb l,2,3-tri-substituted benzenes including 2,6-DCT intended by the present invention as a representative component, while strongly adsorbing l,2,4-tri-substituted benzenes.

Thus, it is possible to selectively and efficiently separate 2,6-DCT of a high purity by using the adsorber of the present invention.

The mixture of DCT isomers to be used in the present invention is a mixture of DCT isomers obtained by chlorination of toluene, which comprises 2,3-DCT (8-l2% by weight), 2,4-DCT (20-35% by weight), 2,5-DCT (25-55% by weight), 2,6-DCT (5-25% by weight) and 3,4-DCT (5-l2% by weight), or a mixture of DCT isomers obtained by chlorination of o-chlorotoluene which comprises 2,3-DCT (5-20% by weight), 2,4-DCT (10-25% by weight), 2,5-DCT (30-70% by weight) and 2,6-DCT (5-30% by weight).

The mixtures of DCT isomers obtained by such chlorinations may be used as they are. Preferably, however, they are firstly separated by rectification into distillation-separable fractions, and the fraction containing 2,6-DCT is subjected to the method of the present invention. Namely, they may be separated into a fraction having a boiling point of about 201 °C comprising 2,4-DCT, 2,5-DCT and 2,6-DCT, and a fraction having a boiling point of from about 208 to 209 °C comprising 2,3-DCT and/or 3,4-DCT, by rectification. The method of the present invention is effective particularly for the separation and recovery of 2,6-DCT from the former fraction of a mixture of DCT isomers.

The ZSM zeolites to be used in the present invention include ZSM-8 as disclosed in e.g. GB-B-1,334,243, ZSM-11 as disclosed in JP-B-23,280/1978, ZSM-21 as disclosed in US-4,001,346, ZSM-35 as disclosed in JP-A-144,500/1978, zeolite Zeta-1 as disclosed in JP-A-67299/1976 and zeolite Zeta-3 as

disclosed in JP-A-67,298/1976.

The TPZ-3 zeolite to be used in the present invention is a high silica content zeolite represented by the following general formula:

$Na_2O.Al_2O_3.xSiO_2$ ($x \geqq 10$)

As expressed in the form of an anhydrous oxide. It shows an X-ray diffraction pattern which is entirely different from conventional ZSM zeolites such as ZSM-5, ZSM-ll, ZSM-l2 and ZSM-38 or from Zeta-3 zeolite, and its properties are also different therefrom. In the above formula, the sodium ions can easily be exchanged by other cations, as is well-known to those skilled in the art of zeolites.

As the cation component of the zeolites to be used in the present invention, any component may basically be used. However, it is preferably at least one cation selected from the group consisting of monovalent and divalent metals, protons and ammonium ions. Particularly preferred are protons.

The ion exchange of these cations may be conducted preferably by contacting to zeolites an aqueous solution of a nitrate of one or more cations to be exchanged with the cations of the zeolites, as an ion exchange treating solution. It is also preferred to employ an aqueous solution of other soluble salts such as chlorides instead of nitrates. Further, such cations may be treated only onec by a single ion exchange treatment with the ion exchange solution, or may be treated several times. The treating system may be a batch system or a continuous system. The temperature for the treatment is usually within a range of from 20 to 100 ° C, and to facilitate the exchange rate, the temperature is preferably from 50 to 100 ° C. After the ion exchange treatment, it is necessary to conduct washing with water adequately so that e.g. $NO_3^-$ or $Cl^-$ ions are no longer detected.

Prior to the use of the zeolite as a catalyst, it is necessary to remove water of crystallization therefrom. Usually, the content of water of crystallization can be substantially reduced at a temperature of 100 ° C or higher. Preferably, the zeolite is heated at a temperature of from 300 to 600 ° C, whereby almost all water of crystallization can be removed.

The zeolite to be used in the present invention may be in the form of a powder, or crushed blocks, or may be shaped products obtained by e.g. compression molding and extrusion molding. Further if necessary, a binder such as alumina sol or clay may be added at the time of the molding. In a small scale operation, the zeolite may be used in a powder form. For an industrial operation, it is preferred to employ a molded product in the form of pellets having a diameter of from 0.1 to 10 mm to avoid a pressure loss. The shape of the zeolite may be suitably selected depending upon the particular apparatus.

The $SiO_2/Al_2O_3$ ratio is not particularly restricted, and is preferably within a range of from 10 to 200. A method for the production of TPZ-3 and its composition are disclosed in JP-A-95821/1982.

Namely, TPZ-3 is prepared by using N,N,N,N',N',N'-hexaalkyl-1,6-hexanediammonium ions, and has characteristic pores larger than ZSM-5. For the operation of the method of the present invention a batch method using a conventional fixed bed system or a continuous method may be used for the separation step.

The separation step of the present invention may be carried out basically by a cycle of adsorption, washing, desorption and regeneration of the adsober, with one or more adsorption chambers packed with the adsorber.

Namely, a mixture of DCT isomers including 2,6-DCT as the desired substance and at least one other DCT isomer except for 2,3-DCT, is contacted in an adsorption chamber with the adsober of the present invention, whereby the desired 2,6-DCT can selectively be separated as a non-adsorbed component while having other components strongly adsorbed on the adsorber.

The adsorption of the present invention may be conducted within a temperature range of from room temperature to 300 ° C, preferably from 150 to 250 ° C. If the temperature is higher than 300 ° C, a side reaction such as a disproportionation reaction of DCT is likely to take place, such being undesirable.

The reaction pressure is usually with in a range of from atmospheric pressure to 50 $kg/cm^2$, preferably from atmospheric pressure to 30 $kg/cm^2$. If the pressure is higher than about 50 $kg/cm^2$, the operation will be costly, such being undesirable. At the time of adsorption, a substance which does not affect the adsorption-desorption, may be added to the mixture of DCT isomers, as a diluting solvent.

There is no particular restriction as to the manner of desorbing DCT isomers strongly adsorbed by the adsorptive separation of the present invention. However, steam desorption is preferably employed.

With respect to the ability of the zeolite to be used in the method of the present invention for the adsorptive separation of the mixture of DCT, for instance, in a case where a mixture comprising 2,4-DCT, 2,5-DCT and 2,6-DCT is subjected to adsorptive separation with ZSM-ll, 2,4-DCT and 2,5-DCT are adsorbed, while desired 2,6-DCT will be separated without being adsorbed. Namely, ZSM-ll has an

extremely large adsorption capacity for 2,4-DCT and 2,5-DCT, and the concentration of 2,6-DCT in the non-adsorbed effluent changes idealy as shown by the break through curve of Figure 1. Thus, the capability of ZSM-ll for adsorptive separation can be represented by the amount of 2,6-DCT effluent (% by weight as pure product) up to the break through point per 1 g of zeolite.

$$\text{2,6-DCT separation capacity (wt\%)} = \frac{A(g) \times B(wt\%)}{\text{ZSM-11 (g)}}$$

A: Total amount (g) of the effluent up to the break through point

B: Average 2,6-DCT concentration (wt%) in the effluent

The higher the 2,6-DCT separation capacity, the more advantageous for the industrial operation, and consequently, it is possible to obtain 2,6-DCT of a high purity efficiently.

Thus, according to the method of the present invention, it is possible not only to selectively obtain 2,6-DCT of a high purity which used to be difficult to accomplish by the adsorptive separation of a mixture of DCT isomers by means of the zeolite, but also to treat other DCT isomers separated as strongly adsorbed components, for isomerization to be used again for adsorptive separation, whereby the respective isomers can effectively be utilized. Further, the zeolites of the present invention can be reused for a long period of time. Thus, the contribution of the present invention to the industrial application is extremely high.

Now, the present invention will be described in further detail with reference to Examples. However, it should be understood that the present invention is by no means restricted to such specific Examples.

Reference Example 1

ZSM-11 zeolite powder having a molar ratio of $SiO_2/Al_2O_3$ = 50.8 was obtained in accordance with the method of Example 1 of JP-B-23280/1978. Then, the powder was subjected to ion exchange five times with a 10 wt% ammonium nitrate aqueous solution (liquid-solid ratio of 2.0 l/kg, 95°C), then thoroughly washed with water, dried at 150°C for 5 hours, and thereafter sintered at 500°C for 3 hours to obtain acid type H-ZSM-11 zeolite powder. The results of the X-ray analysis of this H-ZSM-11 zeolite agreed to those of H-ZSM-11 made by Mobile Company.

Reference Example 2

ZSM-8 zeolite powder was prepared in accordance with the method of the Examples of GB-B-1,334,243.

This powder was treated in the same manner as in Reference Example 1 to obtain H-ZSM-8 zeolite powder.

Reference Example 3

ZSM-21 zeolite powder was prepared in accordance with the method of the Examples of US-4,001,346.

This powder was treated in the same manner as in Reference Example 1 to obtain H-ZSM-21 zeolite powder.

Reference Example 4

ZSM-35 zeolite powder was prepared in accordance with the method of JP-A-144,500/1978.

The powder was treated in the same manner as in Reference Example 1 to obtain H-ZSM-35 zeolite powder.

Reference Example 5

Zeta-1 zeolite powder was prepared in accordance with the method of the Examples of JP-A-67,299/1976.

The powder was treated in the same manner as in Reference Example 1 to obtain H-Zeta-1 zeolite powder.

Reference Example 6

Zeta-3 zeolite powder was prepared in accordance with the method of the Examples of JP-A-67,298/1976.

The powder was treated in the same manner as in Reference Example 1 to obtain H-Zeta-3 zeolite powder.

Example 1

8.48 g of H-ZSM-11 zeolite powder of Reference Example 1 was packed to a metal column having an internal diameter of 9.8 mm and a length of 16.3 cm, and a mixture of DCT isomers was introduced at a rate of 0.1 ml/min. at 200 °C under a nitrogen pressure of 2 $kg/cm^2$. The composition of the mixture of DCT isomers introduced was as follows:

2,4-/2,5-/2,6-DCT = 24.1/43.7/32.2 by weight ratio

The composition of the non-adsorbed effluent from the outlet of the column was analyzed by gas chromatography, whereby it was found that the initial 2,6-DCT concentration was 100%, the 2,6-DCT concentration gradually decreased, and upon expiration of 10 minutes, the composition of the non-adsorbed effluent became the same as the composition of the influent, thus reached the break through point. The total amount of the non-adsorbed effluent up to the break through point was 0.70 g. The average composition of the total effluent was as follows:

2,4-/2,5-/2,6-DCT = 7.2/13.6/79.2 by weight ratio

Thus, the 2,6-DCT separation capacity was 6.54% by weight.

Comparative Examples 1 to 4

The adsorption operation was repeated by using the same apparatus, method and DCT isomer mixture as used in Example l by changing the type of the zeolite . The zeolites used were Na-X zeolite (Molecular Sieve l3X, manufactured by Union Showa K.K.), K-Y zeolite (TSZ-320 KOA, manufactured by Toyo Soda Manufacturing Company Limited), Na-A zeolite (Molecular Sieve 4A, manufactured by Union Showa K.K.), and K-L zeolite (TSZ-500 KOA, manufactured by Toyo Soda Manufacturing Company Limited). 10 g of such zeolite was packed in a metal column. The average composition of DCT in the non-adsorbed effluent up to the break through point is shown in the following Table.

| Comparative Examples | Type of Zeolite | Effluent Composition(wt%) | | | Amount (g) |
|---|---|---|---|---|---|
| | | 2,4-DCT | 2,5-DCT | 2,6-DCT | |
| Comparative Example 1 | Na-X | 30.9 | 43.5 | 25.6 | 1.4 |
| Comparative Example 2 | K-Y | 13.4 | 37.3 | 49.3 | 1.4 |
| Comparative Example 3 | Na-A | 24.0 | 44.0 | 32.0 | No adsorptive separation |
| Comparative Example 4 | K-L | 24.0 | 44.0 | 32.0 | " |

Examples 2 to 5

The adsorption operation was conducted by using the same apparatus and method as used in Example 1 except that the cations of H-ZSM-11 zeolite of Reference Example 1 were changed to calcium, magnesium, copper and sodium, respectively. The 2,6-DCT separation capacity was measured. The results are shown in the following Table.

| Example No. | Type of cations | 2,6-DCT separation capacity (wt%) |
|---|---|---|
| 2 | Ca | 4.95 |
| 3 | Mg | 2.66 |
| 4 | Cu | 4.61 |
| 5 | Na | 4.69 |

The exchange of cations were conducted by treating H-ZSM-II zeolite with a 5-10 wt% nitrate aqueous solution in the same manner as in Reference Example 1.

Examples 6 and 7

The operation was conducted by using the same apparatus and method as used in Example 1 by changing the temperature for adsorption. The 2,6-DCT separation capacity was measured. The results are shown in the following Table. However, when the adsorption temperature was 300 ° C, a disproportionation reaction occured, whereby o-chlorotoluene and toluene were produced as by-products.

| Ex. No. | Adsorption temperature (°C) | Composition of the total effluent up to the break through point | | | | | 2,6-DCT separation capacity (wt%) |
|---|---|---|---|---|---|---|---|
| | | 2,4-DCT | 2,5-DCT | 2,6-DCT | o-chloro-toluene | toluene | |
| 6 | 250 | 7.4 | 13.6 | 79.0 | 0 | 0 | 6.46 |
| 7 | 300 | 7.2 | 13.5 | 75.0 | 3.9 | 0.1 | 5.96 |

Examples 8 to 10

The adsorption operation was conducted by using the same apparatus and method as used in Example 1 by changing the proportions of the mixture of DCT isomers introduced. The composition of the influent and the average composition of the non-adsorbed effluent up to the break through point are shown in the following Table.

| Ex. No. | Influent composition (wt%) | | | | | Effluent composition (wt%) | | | | |
|---|---|---|---|---|---|---|---|---|---|---|
| | 2,3-DCT | 2,4-DCT | 2,5-DCT | 2,6-DCT | 3,4-DCT | 2,3-DCT | 2,4-DCT | 2,5-DCT | 2,6-DCT | 3,4-DCT |
| 8 | - | 40.2 | 36.4 | 23.4 | - | - | 12.1 | 11.7 | 76.2 | - |
| 9 | 14.7 | 22.8 | 32.9 | 29.6 | - | 26.9 | 9.0 | 13.3 | 50.8 | - |
| 10 | 20.1 | 14.5 | 25.4 | 20.4 | 19.6 | 36.1 | 5.4 | 9.8 | 41.4 | 7.3 |

Examples 11 to 15

The adsorption operation was conducted by using the same apparatus and method and DCT isomer mixture as used in Example 1 except that H-ZSM-II zeolite used in Example 1 was changed to H-ZSM zeolite powder prepared in Reference Examples 2 to 6. The 2,6-DCT separation capacity was measured. The results are shown in the following Table.

| Ex. No. | Type of ZSM zeolite | | Effluent composition (wt%) | | | Separating capacity (wt%) |
|---|---|---|---|---|---|---|
| | Name | Reference Example No. | 2,4-DCT | 2,5-DCT | 2,6-DCT | |
| 11 | H-ZSM-8 | 2 | 10.8 | 19.8 | 69.4 | 5.48 |
| 12 | H-ZSM-21 | 3 | 10.3 | 18.9 | 70.8 | 5.65 |
| 13 | H-ZSM-35 | 4 | 8.8 | 16.3 | 74.9 | 5.96 |
| 14 | H-Zeta-1 | 5 | 7.5 | 13.7 | 78.8 | 6.37 |
| 15 | H-Zeta-3 | 6 | 11.3 | 21.1 | 67.6 | 5.42 |

Reference Example 7

TPZ-3 zeolite powder having a molar ratio of $SiO_2/Al_2O_3$ = l00 was prepared in accordance with the method of Example 1 of Japanese Unexamined Patent Publication No. 9582l/l982.

Then, the powder was treated in the same manner as in Reference Example 1, to obtain acid type H-TPZ-3 zeolite powder. The results of the X-ray analysis of this H-TPZ-3 zeolite agreed to those of H-TPZ-3

manufactured by Teijin Yuka K.K.

Reference Example 8

TPZ-3 zeolite powder having a molar ratio of $SiO_2/Al_2O_3$ = 120 was prepared in the same manner as in Reference Example 7.

The powder was treated in the same manner as in Reference Example 1 to obtain H-TPZ-3 zeolite powder.

Examples 16 to 19

8.43 g of H-TPZ-3 zeolite powder of Reference Example 7 was packed in a metal column having an internal diameter of 9.8 mm and a length of 16.3 cm, and a mixture of DCT isomers was introduced at a rate of 0.1 mm/min. at 200 °C under a nitrogen pressure of 2 $kg/cm^2$. The composition of the mixture of DCT isomers introduced was as follows:

2,4-/2,5-/2,6-DCT = 24.1/43.7/32.2 by weight ratio

The composition of the non-adsorbed effluent from the outlet of the column was analyzed by gas chromatography, whereby it was found that the initial 2,6-DCT concentration was l00%, and the 2,6-DCT concentration gradually decreased, and upon expiration of 10 minutes, the composition of the non-adsorbed effluent became the same as the composition of the influent, and thus reached the break through point.

The total amount of the non-adsorbed effluent up to the break through point was 0.70 g.

The average composition of DCT in the total effluent was as follows:

2,4-/2,5-/2,6-DCT = 7.5/14.0/78.5 by weight ratio

Thus, the 2,6-DCT separation capacity was 6.52% by weight. Then, nitrogen gas was introduced at the same temperature under a pressure of 3 $kg/cm^2$ for 30 minutes, to discharge and wash the deposited mixture of DCT isomers. The discharged amount was 1.2 g.

Then, a gas mixture of steam (molar ratio: 0.33) and nitrogen (molar ratio: 0.67) was introduced at a rate of 60 mm/min. at the same temperature under a pressure of 6 $kg/cm^2$. The adsorbed 2,4-DCT and 2,5-DCT were desorbed and discharged together with water, and upon expiration of about 30 minutes, the discharge of DCT was completed. The total amount of the desorbed DCT effluent was 0.28, and the average composition of the total effluent was as follows:

2,4-/2,5-/2,6-DCT = 31.4/57.8/10.8 by weight ratio

Further, a nitrogen gas was introduced at a rate of 40 ml/min. at the same temperature under a pressure of 6 $kg/cm^2$ for 2 hours to dry and regenerate the adsorber.

After the completion of the regeneration, a total of 4 cycles including Example 16 were repeated, with one cycle comprising the above adsorption-washing-desorption-regeneration steps. The results are shown in the following Table. The crystallinity of the zeolite after the repetition of the 4 cycles was analyzed by the X-ray analysis, and no destruction the crystal structure was observed.

| Ex. Cycle No. | Effluent composition during the adsorption step (wt%) | | | Total effluent during the adsorption step (g) | Separation capacity (wt%) |
|---|---|---|---|---|---|
| | 2,4-DCT | 2,5-DCT | 2,6-DCT | | |
| 17 2nd | 8.9 | 16.4 | 74.7 | 0.65 | 5.76 |
| 18 3rd | 8.7 | 16.2 | 75.1 | 0.64 | 5.70 |
| 19 4th | 8.9 | 16.4 | 74.4 | 0.66 | 5.85 |

Examples 20 to 23

The adsorption operation was conducted by using the same apparatus and method as used in Example 16 except that the cations of H-TPZ-3 zeolite of Reference Example 7 were changed to calcium, magnesium, copper and sodium, respectively. The 2,6-DCT separation capacity was measured. The results are shown in the following Table.

| Example No. | Type of cations | 2,6-DCT separation capacity (wt%) |
|---|---|---|
| 20 | Ca | 5.20 |
| 21 | Mg | 2.65 |
| 22 | Cu | 4.79 |
| 23 | Na | 4.69 |

The cation exchange was conducted by treating H-TPZ-3 zeolite with a 5-10 wt% nitrate aqueous solution in the same manner as in Reference Example 1.

Examples 24 and 25

The operation was conducted by using the same apparatus and method as used in Example 16 by changing the temperature for adsorption. The 2,6-DCT separation capacity was measured. The results are shown in the following Table. However, when the adsorption temperature was 300 ° C, a disproportionation reaction took place, whereby o-chlorotoluene and toluene were produced as by-products.

| Ex. No. | Adsorption temperature (°C) | Composition of the total effluent up to the break through point | | | | | 2,6-DCT separation capacity (wt%) |
|---|---|---|---|---|---|---|---|
| | | 2.4-DCT | 2,5-DCT | 2,6-DCT | o-chloro-toluene | toluene | |
| 24 | 250 | 7.7 | 14.2 | 78.1 | 0 | 0 | 6.46 |
| 25 | 300 | 7.4 | 13.5 | 74.9 | 4.1 | 0.1 | 6.08 |

Examples 26 to 28

The adsorption operation was conducted by using the same apparatus and method as used in Example

16 by changing the proportions of the mixture of DCT isomers introduced. The composition of the influent and the average composition of the non-adsorbed effluent up to the break through point are shown in the following Table.

| Ex. No. | Influent composition (wt%) | | | | | Effluent composition (wt%) | | | | |
|---|---|---|---|---|---|---|---|---|---|---|
| | 2,3-DCT | 2,4-DCT | 2,5-DCT | 2,6-DCT | 3,4-DCT | 2,3-DCT | 2,4-DCT | 2,5-DCT | 2,6-DCT | 3,4-DCT |
| 26 | - | 40.2 | 36.4 | 23.4 | - | - | 12.1 | 11.5 | 76.4 | - |
| 27 | 14.7 | 22.8 | 32.9 | 29.6 | - | 26.2 | 9.3 | 14.1 | 50.4 | - |
| 28 | 20.1 | 14.5 | 25.4 | 20.4 | 19.6 | 30.9 | 6.8 | 11.9 | 42.5 | 7.9 |

Example 29

The operation was conducted in the same manner as in Example 16 except that 8.19 g of H-TPZ-3 zeolite powder of Reference Example 8 was used. The following results were obtained.

Total amount of non-adsorbed effluent up to the break through point: 0.69 g.

The average composition of DCT in this total effluent was as follows:

2,4-/2,5-/2,6-DCT = 7.4/14.0/78.6 by weight ratio

Thus, the 2,6-DCT separation capacity was 6.62% by weight.

**Claims**

**1.** A method for selectively separating 2,6-dichlorotoluene from a mixture of dichlorotoluene isomers by means of a zeolite adsorber, characterized in that a zeolite selected from the group consisting of ZSM-8 zeolite, ZSM-11 zeolite, ZSM-21 zeolite, ZSM-35 zeolite, Zeta-1 zeolite, Zeta-3 zeolite and TPZ-3 zeolite, is used as the adsorber to selectively separate 2,6-dichlorotoluene as a non-adsorbed component.

**2.** The method according to Claim 1, wherein said mixture is a mixture of 2,4-, 2,5- and 2,6-dichlorotoluenes.

**3.** The method according to Claim 1, wherein the zeolite has cations selected from monovalent and divalent metals, protons and ammonium ions.

**4.** The method according to Claim 1, wherein TPZ-3 zeolite has the formula:

$Na_2OAl_2O_3.xSiO_2$ ($x \geqq 10$)

as expressed in the form of an anhydrous oxide.

**5.** The method according to Claim 1, wherein the zeolite contains no substantial water of crystallization.

**6.** The method according to Claim 1, wherein the zeolite is in the form of pellets having an average diameter of from 0.1 to 10 mm.

7. The method according to Claim 1, wherein the $SiO_2/Al_2O_3$ ratio in the zeolite is from 10 to 200.

8. The method according to Claim 1, wherein the adsorption is conducted at a temperature of from room temperature to 300 ° C.

9. The mothod according to Claim 1, wherein the adsorption is conducted at a pressure of from atmospheric pressure to 50 $kg/cm^2$.

**Patentansprüche**

1. Verfahren zur selektiven Abtrennung von 2,6-Dichlortoluol aus einer Mischung von Dichlortoluolisomeren mittels eines Zeolith-Adsorbers, dadurch gekennzeichnet, daß ein Zeolith, ausgewählt aus der Gruppe, bestehend aus ZSM-8-Zeolith, ZSM-11-Zeolith, ZSM-21-Zeolith, ZSM-35-Zeolith, Zeta-1-Zeolith, Zeta-3-Zeolith und TPZ-3-Zeolith als der Adsorber verwendet wird, um 2,6-Dichlortoluol in selektiver Weise als eine nicht-adsorbierte Komponente abzutrennen.

2. Verfahren gemäß Anspruch 1, wobei die Mischung eine Mischung von 2,4-, 2,5- und 2,6-Dichlortoluolen ist.

3. Verfahren gemäß Anspruch 1, wobei der Zeolith Kationen aufweist, die ausgewählt sind unter monovalenten und divalenten Metallen, Protonen und Ammoniumionen.

4. Verfahren gemäß Anspruch 1, wobei TPZ-3-Zeolith die folgende Formel aufweist:

$Na_2OAl_2O_3.xSiO_2$ $(x \geqq 10)$

ausgedrückt in Form eines wasserfreien Oxids.

5. Verfahren gemäß Anspruch 1, wobei der Zeolith im wesentlichen kein Kristallwasser enthält.

6. Verfahren gemäß Anspruch 1, wobei der Zeolith in der Form von Pellets vorliegt, welche einen durchschnittlichen Durchmesser von 0,1 bis 10 mm aufweisen.

7. Verfahren gemäß Anspruch 1, wobei das $SiO_2/Al_2O3$-Verhältnis in dem Zeolith von 10 bis 200 beträgt.

8. Verfahren gemäß Anspruch 8, wobei die Adsorption bei einer Temperatur von Zimmertemperatur bis 300 ° C durchgeführt wird.

9. Verfahren gemäß Anspruch 1, wobei die Adsorption bei einem Druck von atmosphärischem Druck bis 50 $kg/cm^2$ durchgeführt wird.

**Revendications**

1. Procédé pour la séparation sélective du dichloro-2,6 toluène à partir d'un mélange d'isomères du dichlorotoluène, à l'aide d'un adsorbant en zéolithe, caractérisé par le fait qu'on utilise, comme adsorbant, une zéolithe choisie dans le groupe constitué par la zéolithe ZSM-8, la zéolithe ZSM-11, la zéolithe ZSM-21, la zéolithe ZSM-35, la zéolithe Zéta-1, la zéolithe Zéta-3 et la zéolithe TPZ-3, pour séparer de façon sélective le dichloro-2,6 toluène en tant que composant non-adsorbé.

2. Procédé selon la revendication 1, dans lequel ledit mélange est un mélange de dichloro-2,4, -2,5 et -2,6 toluènes.

3. Procédé selon la revendication 1, dans lequel la zéolithe présente des cations choisis parmi les métaux monovalents et divalents, les protons et les ions ammonium.

4. Procédé selon la revendication 1, dans lequel la zéolithe du type TPZ-3 a la formule :

$Na_2OAl_2O_3 \cdot xSiO_2$ $(x \geqq 10)$

telle qu'exprimée sous la forme d'un oxyde anhydre.

5. Procédé selon la revendication 1, dans lequel la zéolithe ne contient pratiquement pas d'eau de cristallisation.

6. Procédé selon la revendication 1, dans lequel la zéolithe se présente sous la forme de boulettes ayant un diamètre moyen de 0,1 à 10 mm.

7. Procédé selon la revendication 1, dans lequel le rapport $SiO_2/Al_2O_3$ dans la zéolithe est de 10 à 200.

8. Procédé selon la revendication 1, dans lequel l'adsorption est effectuée à une température située dans la plage allant de la température ambiante à 300 °C.

9. Procédé selon la revendication 1, dans lequel l'adsorption est conduite à une pression située dans la plage allant de la pression atmosphérique à 50 $kg/cm^2$.

# FIGURE 1

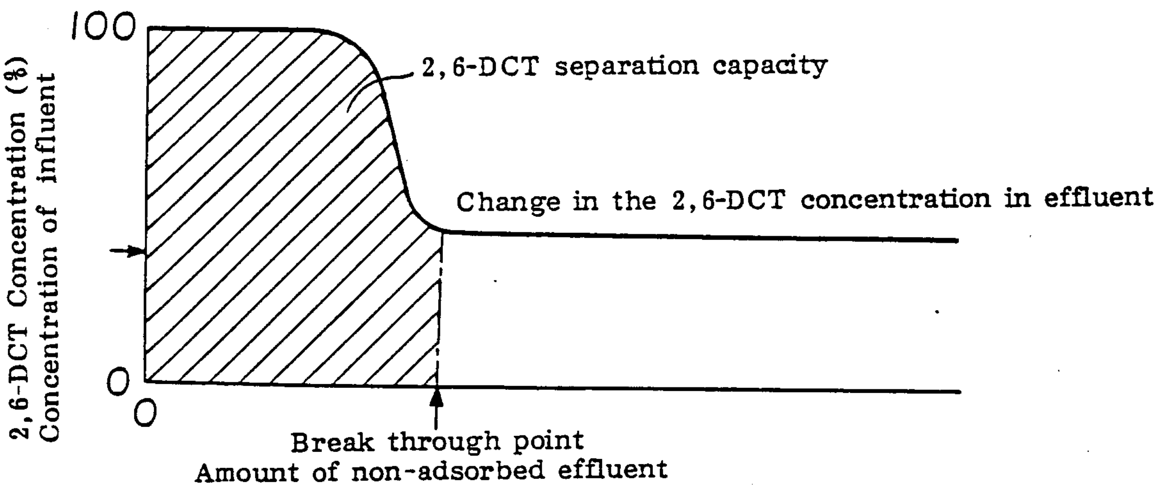

100
0
0
2,6-DCT Concentration (%)
Concentration of influent
2,6-DCT separation capacity
Change in the 2,6-DCT concentration in effluent
Break through point
Amount of non-adsorbed effluent